Europäisches Patentamt

European Patent Office

Office européen des brevets

(19)

(11) Veröffentlichungsnummer : **0 074 021**
**B1**

# (12) EUROPÄISCHE PATENTSCHRIFT

(45) Veröffentlichungstag der Patentschrift :
21.11.85

(21) Anmeldenummer : 82107827.6

(22) Anmeldetag : 25.08.82

(51) Int. Cl.⁴ : **A 61 B  6/06, G 21 K  1/04**

(54) **Röntgenuntersuchungsgerät.**

(30) Priorität : 07.09.81 DE 3135421

(43) Veröffentlichungstag der Anmeldung :
16.03.83 Patentblatt 83/11

(45) Bekanntmachung des Hinweises auf die Patenterteilung : 21.11.85 Patentblatt 85/47

(84) Benannte Vertragsstaaten :
DE FR

(56) Entgegenhaltungen :
EP-A- 0 024 028
FR-A- 2 482 444
US-A- 3 780 291
US-A- 4 031 401
US-A- 4 266 135
PATENTS ABSTRACTS OF JAPAN, Band 1, Nr. 152, 7.
Dezember 1977, Seite 8032E77

(73) Patentinhaber : Siemens Aktiengesellschaft
Berlin und München Wittelsbacherplatz 2
D-8000 München 2 (DE)

(72) Erfinder : Arauner, Alfred
Keplerstrasse 47
D-8510 Fürth (DE)

## Beschreibung

Die Erfindung bezieht sich auf ein Röntgenuntersuchungsgerät mit einer Patientenlagerungsplatte, mit einer auf der einen Seite der Patientenlagerungsplatte angeordneten Röntgenröhre, mit einer ein eng ausgeblendetes in einer Ebene fächerförmig schwenkbares Röntgenstrahlenbündel erzeugenden Primärstrahlenausblendvorrichtung und mit einer auf der anderen Seite der Patientenlagerungsplatte angeordneten, dem Strahlenbündel ausgesetzten Detektoranordnung.

Es ist bereits ein Röntgenuntersuchungsgerät bekannt geworden, bei dem der aus einer Röntgenröhre austretende Strahlenkegel durch eine Schlitzblende fächerförmig eingeblendet wird und bei dem in Strahlenrichtung hinter der Schlitzblende eine radial geschlitzte kreisförmige Blendenscheibe aus strahlenundurchlässigem Material in einer Ebene senkrecht zur Strahlenrichtung drehbar gelagert ist (US-A-3 780 291). Das so ausgeblendete, etwa bleistiftstarke Strahlenbündel bewegt sich beim Drehen der Blendenscheibe in der Fächerebene jeweils von der einen zu der anderen Seite. Diesem Strahlenbündel ist hinter dem Untersuchungsobjekt eine Detektoranordnung zugeordnet. Beim Verschieben der Röntgenuntersuchungseinrichtung senkrecht zur Fächerebene läßt sich eine Scheibe des Untersuchungsobjektes nach der anderen abtasten. Die Meßwerte der einzelnen zeilenweise angeordneten Detektoren lassen sich speichern und auf einem Fernsehsichtgerät zeilenweise in verschiedenen Graustufen abbilden. Das Bild entspricht einer konventionellen Röntgenaufnahme. Jedoch ist der Streustrahlenanteil wegen des jeweils relativ kleinen, von Röntgenstrahlen durchsetzten Volumens gering. Es ist eine Eigenart dieser Anordnung, daß sich die Form des ausgeblendeten Strahlenbündels während des Drehens der Blendenscheibe über der schlitzartigen Ausblendung ändert. Auch muß die Blendenscheibe einen deutlich größeren Durchmesser haben als die Breite des ausgeblendeten Strahlenfächers in der Ebene der Blendenscheibe. Dies führt dazu, daß die gesamte Ausblendanordnung so voluminös wird, daß sie nur bei Untertischröhrengeräten einsetzbar ist. Aber auch dort zeigt sich, daß beim Verschieben in Tischlängsrichtung nur ein Teil des Längshubes ausnutzbar ist, weil anderenfalls die geschlitzte Blendenscheibe an der Stirnseite des Tischgestelles anstoßen würde.

Es ist ferner bekannt, ein Untersuchungsobjekt in einer Ebene durch einen dünnen Röntgenstrahl abzutasten, der über die Ebene geschwenkt wird (JP-A-52 93 384). Für die Bewegung des Röntgenstrahles ist dabei eine Ausblendvorrichtung vorgesehen, die einen mit seiner Symmetrieachse etwa senkrecht zur Strahlenrichtung ausgerichteten, gleichmäßig angetriebenen Hohlzylinder enthält, dessen strahlenundurchlässige Wandung zwei spiralförmige Schlitze trägt. Über die konstruktive Ausgestaltung eines Gerätes zur Abtastung des Untersuchungsobjektes ergibt sich aus dieser Schrift jedoch nichts. Insbesondere läßt sich aus dieser Schrift nicht entnehmen, wie die Röntgenstrahlung in Strahlenrichtung gesehen vor dem Zylinder gebündelt und geformt werden soll.

Schließlich zeigt die US-A-4 266 135 einen Computertomographen, bei dem ein fächerförmiges Strahlenbündel zur Durchstrahlung des Untersuchungsobjektes benutzt wird, das auch aus fingerförmig auseinanderlaufenden Einzelstrahlen bestehen kann. Das fächerförmige Strahlenbündel ist dabei durch eine Blende in seiner Breite veränderbar. Aus dieser Schrift ergibt sich jedoch nicht die Abtastung eines Untersuchungsobjektes durch einen einzigen über dieses Objekt bewegten Strahl.

Der Erfindung liegt die Aufgabe zugrunde, den Untersuchungsbereich bei Röntgenuntersuchungsgeräten, die das Untersuchungsobjekt mit einem eng ausgeblendeten Röntgenstrahl zeilenförmig abtasten, zu vergrößern. Auch soll eine konstruktive Lösung gefunden werden, die es gestattet, bei einem solchen Röntgenuntersuchungsgerät unter Umkehrung der Strahlenrichtung mit einer Obertischröntgenröhre und einer Untertischaufnahmeeinrichtung zu arbeiten.

Bei einem Röntgenuntersuchungsgerät der eingangs genannten Art ist diese Aufgabe erfindungsgemäß dadurch gelöst, daß die Ausblendvorrichtung einen mit seiner Symmetrieachse etwa senkrecht zur Strahlenrichtung ausgerichteten, gleichmäßig angetriebenen Hohlzylinder enthält, dessen strahlenundurchlässige Wandung zwei um 180° gegeneinander versetzte, spiralförmige Schlitze trägt, daß der Hohlzylinder zwischen der Strahlenquelle und der Patientenlagerungsplatte fokusfern am Ende einer trichterartigen, dem fächerförmigen Strahlenbündel entsprechenden Halterung gelagert ist, deren Querschnitt durch den Hohlzylinder ausgefüllt ist, und daß in der durch den Fokus und die Symmetrieachse des Hohlzylinders gegebenen Ebene eine zusätzliche fokusnahe schlitzförmige Einblendung angeordnet ist. Ein solcher Hohlzylinder läßt die Strahlung nur in dem Bereich durch, in dem sich die beiden Schlitze auf der in Strahlenrichtung vorderen und hinteren Seite der Mantelfläche in einer Linie mit dem Fokus befinden. Dies ist stets nur in einem einzigen Punkt der Fall. Dieser Punkt wandert bei der Drehung des Hohlzylinders in axialer Richtung. Das ausgeblendete Strahlenbündel hat einen rhombenförmigen Querschnitt. Es ist ein größer Vorteil dieser Konstruktion, daß die Ausblendvorrichtung nur wenig Platz senkrecht zur Fächerebene benötigt. Bei der Verwendung mit einer Untertischröntgenröhre wird so der Längshub unterhalb des Tischgestelles nicht behindert. Aus demselben Grund eignet sich diese Ausblendvorrichtung auch besonders für Röntgenuntersuchungsgeräte, bei denen sich die Röntgenröhre mitsamt der Ausblendvorrichtung oberhalb und die Detektoren unterhalb der Patientenlagerungsplatte befinden. Die erfingungsgemäße Konstruktion verringert die Halbschattenbildung und verhindert zugleich das Austreten von Streustrahlung im Bereich der Ausblendvorrichtung. Die zusätzliche fokusnahe schlitzförmige Einblendung verringert dabei die Streustrahlung noch weiter und steigert die Schärfe des ausgeblendeten Strahlenbündels.

Die Ausblendvorrichtung läßt sich auch für größere Fächerwinkel verwenden, wenn die Schlitzbreite in besonders vorteilhafter Weiterbildung der Erfindung von der Schlitzmitte zu ihren Enden hin verbreitert ist. Dies hat den Vorteil, daß auch bei der an seinen beiden Enden schrägen Durchstrahlung des Hohlzylinders der unterschiedlich axial verschobene Auftreffpunkt des Strahlenbündels auf der der Strahlenquelle zugewandten und der von ihr abgewandten Seite des Hohlzylinders zu keiner Verschmälerung des durchgelassenen Strahlenbündels führt.

Die Strahlenbelastung des Patienten kann noch weiter verringert werden, wenn in besonders zweckmäßiger Weiterbildung der Erfindung eine Blendenplatte in den Strahlengang zwischen Röntgenröhre und Patientenlagerungsplatte eingesetzt ist, die auf die einzelnen Detektorelemente der Detektoranordnung ausgerichtete Löcher trägt. Hierdurch wird jedes einzelne Detektorelement für sich gesondert bestrahlt, währen die Zwischenzonen geschont werden.

Weitere Einzelheiten der Erfindung werden anhand eines in den Figuren dargestellten Ausführungsbeispieles erläutert. Es zeigen :

Figur 1 eine schaubildliche Ansicht des Röntgenuntersuchungsgerätes mit einer erfindungsgemäßen Primärstrahlenausblendvorrichtung,

Figur 2 eine Aufsicht auf eine in der Ausblendvorrichtung einsetzbare fokusnahe Schlitzblende,

Figur 3 eine Darstellung der Abwicklung des Hohlzylinders der Primärstrahlenausblendvorrichtung, und

Figur 4 eine Aufsicht auf eine in der Ausblendvorrichtung einsetzbare gelochte Blendenplatte.

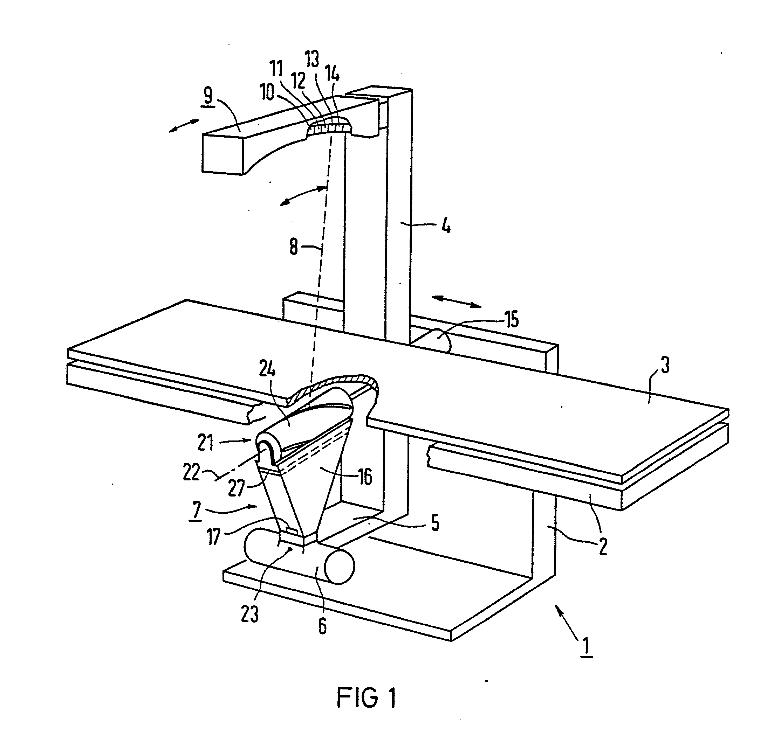

Die Fig. 1 läßt den Aufbau des Röntgenuntersuchungsgerätes 1 erkennen. Auf einem Tischgestell 2 lagert eine gut strahlendurchlässige Patientenlagerungsplatte 3. Sie ist in Tischlängsrichtung verschiebbar. An der in der Darstellung der Fig. 1 hinteren Längsseite des Tischgestells 2 ist eine Stativsäule 4 in Tischlängsrichtung verschiebbar gelagert. Die Stativsäule trägt unter der Patientenlagerungsplatte 3 an einem horizontalen Tragarm 5 eine Röntgenröhre 6. An dieser ist die erfindungsgemäße Ausblendvorrichtung 7 befestigt. Die Stativsäule 4 trägt oberhalb der Patientenlagerungsplatte 3 eine, dem aus der Ausblendvorrichtung austretenden Strahlenbündel 8 ausgesetzte Detektoranordnung 9, mit in einer Reihe dicht nebeneinander angeordneter Einzeldetektoren 11, 12, 13, 14.

Außerdem ist die Stativsäule 4 über einen Antriebsmotor 15 mit konstanter Geschwindigkeit längs der Patientenlagerungsplatte 3 verschiebbar.

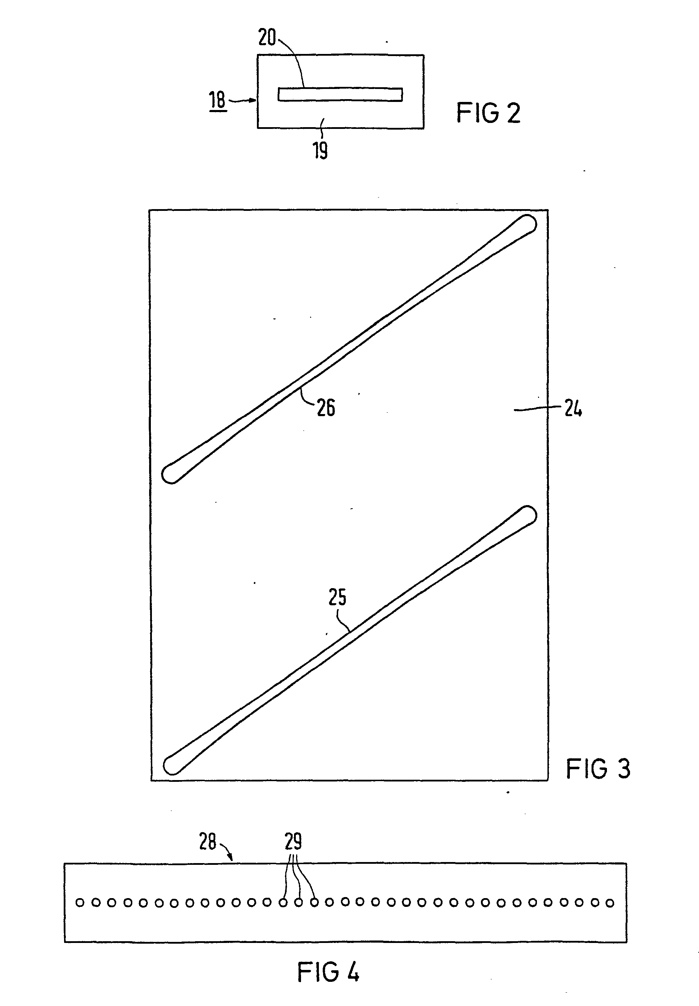

Die Ausblendvorrichtung 7 besitzt eine am Röhrenflansch befestigte tubusartige Halterung 16, die an ihrem fokusnahen Ende eine, im Ausführungsbeispiel durch einen Einschubschlitz 17 einsetzbare Schlitzblende 18 trägt. Diese, in der Fig. 2 in Aufsicht dargestellte, Schlitzblende 18 besteht aus einer einfachen rechteckigen Platte 19 aus strahlenundurchlässigem Material, die mit einem Längsschlitz 20 konstanter Breite versehen ist. Entsprechend dem aus der Schlitzblende 18 austretenden fächerförmigen Strahlenkegel hat auch die tubusartige Halterung 16 einen schmalen, rechteckigen, fächerförmigen Querschnitt. An dem der Schlitzblende 18 abgewandten fokusfernen Ende der tubusartigen Halterung 16 ist ein Hohlzylinder 21 dergestalt an der tubusartigen Halterung drehbar gelagert, daß seine Symmetrieachse 22 genau in der Ebene liegt, die durch den Schlitz 20 der Schlitzblende 18 und dem Fokus 23 der Röntgenröhre 6 gebildet wird. Außerdem ist die Symmetrieachse 22 des Hohlzylinders 21 in etwa senkrecht zur Strahlenrichtung ausgerichtet. Der Durchmesser des Hohlzylinders 21 — im Ausführungsbeispiel etwa 15 cm — füllt den Querschnitt der tubusartigen Halterung 16 vollständig aus. Der Übersichtlichkeit halber ist die tubusartige Halterung im Ausführungsbeispiel der Fig. 1 nur bis an den Hohlzylinder 21 herangeführt dargestellt. Es könnte zweckmäßig sein, die tubusartige Halterung über den Hohlzylinder hinaus bis unmittelbar unter die Patientenlagerungsplatte zu verlängern.

Der Mantel 24 des Hohlzylinders 21 besitzt, wie die Fig. 1 zeigt, zwei spiralförmige Schlitze 25, 26. Ihre Form ist der in der Fig. 2 gezeigten Abwicklung des Mantels 24 des Hohlzylinders 21 zu entnehmen. Der Mantel des Hohlzylinders besteht aus einer strahlenundurchlässigen Platte, in der die beiden Schlitze 25, 26 in einem Winkel von etwa 45° zur seitlichen Begrenzung, hintereinander angeordnet sind. Die beiden Schlitze sind an ihren beiden Enden jeweils etwas keulenartig verbreitert (Fig. 3).

Der Hohlzylinder wird von dem Antriebsmotor 15 gleichmäßig angetrieben. Dieser verschiebt zugleich auch über ein Getriebe die Stativsäule 4 relativ zur Patientenlagerungsplatte 3. Diese Verschiebung kann so gewählt werden, daß der Antriebsmotor die Stativsäule jeweils nach einer vollen Umdrehung des Hohlzylinders 21 um die Breite des Strahlenbündels 8 verschiebt. Die Drehzahl des Antriebsmotors je Zeiteinheit soll. zur Anpassung an die Röhrenleistung variiert werden können.

Beim Einschalten der Röntgenröhre wird ein fächerförmiges Strahlenbündel 8 durch die fokusnahe Schlitzblende 18 ausgeblendet. Dieses fächerförmige Strahlenbündel trifft auf der anderen Seite der tubusartigen Halterung auf den dort drehbar gelagerten Hohlzylinder 21 in der Ebene seiner Symmetrieachse 22 auf. Die Röntgenstrahlung kann den Hohlzylinder nur in demjenigen Bereich der beiden Schlitze 25, 26 durchdringen, in dem sich der Schlitz auf der dem Fokus abgewandten Seite des Hohlzylinders und der Schlitz auf der dem Fokus zugewandten Seite des Hohlzylinders in einer geraden Linie mit dem Fokus befinden oder anders ausgedrückt sich aus der Sicht des Fokus überdecken. Dies ist nur in einem schmalen rhombusförmigen Flächenbereich der Fall. Das so ausgeblendete Strahlenbündel 8 durchdringt die Patientenlagerungsplatte 3, das auf der Patientenlagerungsplatte 3 liegende Untersuchungsobjekt und trifft dann auf die Detektoranordnung 9 auf.

Die fokusnahe Schlitzblende 18 verringert lediglich die zwischen Schlitzblende und Hohlzylinder entstehende Streustrahlung und damit den Streustrahlenanteil der durch die sich überschneidenden Schlitze 25, 26 auf den beiden Seiten des Hohlzylinders hindurchtreten kann. Der Schlitz der fokusnahen Schlitzblende ist daher auch etwas weiter gehalten als das durch den Hohlzylinder durchgehende Strahlenbündel 8. Beim Drehen des Hohlzylinders wandert das Strahlenbündel 8 längs der Symmetrieachse 22 des Hohlzylinders 21. Die Richtung in der das Strahlenbündel 8 wandert, ist durch die Drehrichtung des Hohlzylinders vorgegeben. Dabei überstreicht das Strahlenbündel während jeder halben Umdrehung des Hohlzylinders die gesamte Ausdehnung der Detektoranordnung 9, d. h. die in einer Reihe angeordneten Einzeldetektoren 10, 11, 12, 13, 14. Bei gleichzeitigem Verschieben der Stativsäule läßt sich das Untersuchungsobjekt Scheibe für Scheibe abtasten.

Durch zeilenweises Aufzeichnen der Meßwerte der einzelnen Detektoren 10, 11, 12, 13, 14 der Detektoranordnung 9 auf dem Leuchtschirm eines Fernsehsichtgerätes (nicht dargestellt) läßt sich ein Durchleuchtungsbild des Untersuchungsobjektes konstruieren, das einer herkömmlichen Röntgenaufnahme entspricht. Wegen des geringen durchstrahlten Volumens ist jedoch der Streustrahlenanteil kleiner und der Kontrast größer als bei herkömmlichen Röntgenaufnahmen. Die Zeichenschärfe wächst umgekehrt proportional mit dem Durchmesser des ausgeblendeten Strahlenbündels 8 und dem Abstand der einzelnen Strahlendetektoren voneinander. Sie läßt sich erhöhen, wenn der Vorschub der Stativsäule bei einer halben Umdrehung des Hohlzylinders 21 nur einem halben statt einem ganzen Durchmesser des ausgeblendeten Strahlenbündels entspricht. In diesem Fall überlappt sich jede abgetastete Scheibe des Untersuchungsobjektes mit der nachfolgenden Scheibe um eine halbe Strahlenbündelbreite. Eine weitere Steigerung der Zeichenschärfe läßt sich erreichen, wenn die Detektoranordnung nach jeder abgetasteten Zeile um eine halbe Detektorbreite in Richtung der Detektorzeile verschoben wird.

Im Ausführungsbeispiel ist in der tubusartigen Halterung 16 in unmittelbarer Nachbarschaft zum Hohlzylinder 21 ein weiterer Einschubschlitz 27 für eine in der Fig. 4 dargestellte gelochte Blendenplatte 28 vorgesehen. Diese gelochte Blendenplatte besteht aus einem strahlenundurchlässigen Material und trägt in der Ebene des von der fokusnahen Schlitzblende ausgeblendeten Strahlenfächers eine Reihe von Löchern 29. Diese Löcher sind so angeordnet, daß ein jedes von ihnen bei völlig eingeschobener Blendenplatte 28 zu einem Detektorelement 10, 11, 12, 13, 14 der Detektoranordnung 9 ausgerichtet ist. Hierdurch wird sichergestellt, daß das Strahlenbündel 8 beim Überstreichen der Detektoranordnung 9 in dem Bereich zwischen zwei Detektoren ausgeblendet wird und nur jene Röntgenquanten den Körper des Untersuchungsobjektes durchsetzen, die später auch zur Bildinformation beitragen.

Es ist auch möglich, die in Fig. 4 dargestellte gelochte Blendenplatte auf der dem Fokus abgewandten Seite des Hohlzylinders 21 anzuordnen. Hierdurch wird die Halbschattenbildung wegen der größeren Fokusferne der gelochten Blendenplatte 28 verringert. Ist eine lineare Verschiebung der Detektoren um eine halbe Detektorbreite nach jeder abgetasteten Zeile vorgesehen, so muß auch die gelochte Blendenplatte synchron mit den Detektoren um einen halben Lochabstand verschoben werden. Durch Änderung der Drehzahl des Antriebsmotors 15 läßt sich die Signalhöhe der einzelnen Detektoren 10, 11, 12, 13, 14 der Detektoranordnung an die Dosisleistung der Röntgenröhre 6 anpassen. Dabei ist es auch möglich, mit dem Antriebsmotor statt der Stativsäule 4 die Patientenlagerungsplatte 3 mit dem Untersuchungsobjekt zu verschieben. Die Ausblendvorrichtung läßt sich in ihren Abmessungen verglichen mit der vorbekannten Lösung so klein bauen, daß damit ohne weiteres eine Umkehrung der Strahlenrichtung möglich wird, wenn die Röntgenröhre mit der Ausblendvorrichtung oberhalb der Patientenlagerungsplatte und die Detektoranordnung unterhalb der Patientenlagerungsplatte an der Stativsäule 4 befestigt wird.

**Patentansprüche**

1. Röntgenuntersuchungsgerät mit einer Patientenlagerungsplatte (3), mit einer auf der einen Seite der Patientenlagerungsplatte (3) angeordneten Röntgenröhre (6), mit einer ein eng ausgeblendetes, in einer Ebene fächerförmig schwenkbares Röntgenstrahlenbündel (8) erzeugenden Primärstrahlausblendvorrichtung (7) und mit einer auf der anderen Seite der Patientenlagerungsplatte (3) angeordneten, dem Strahlenbündel (8) ausgesetzten Detektoranordnung (9), dadurch gekennzeichnet, daß die Ausblendvorrichtung (7) einen mit seiner Symmetrieachse (22) etwa senkrecht zur Strahlenrichtung ausgerichteten, gleichmäßig angetriebenen Hohlzylinder (21) enthält, dessen strahlenundurchlässige Wandung (24) zwei um 180° gegeneinander versetzte, spiralförmige Schlitze (25, 26) trägt, daß der Hohlzylinder (21) zwischen der Strahlenquelle (23) und der Patientenlagerungsplatte (3) fokusfern am Ende einer trichterartigen, dem fächerförmigen Strahlenbündel entsprechenden Halterung (16) gelagert ist, deren Querschnitt durch den Hohlzylinder (21) ausgefüllt ist, und daß in der durch den Fokus (23) und die Symmetrieachse (22) des Hohlzylinders (21) gegebenen Ebene eine zusätzliche fokusnahe schlitzförmige Einblendung (18, 20) angeordnet ist.

2. Röntgenuntersuchungsgerät nach Anspruch 1, dadurch gekennzeichnet, daß die Schlitzbreite von der Schlitzmitte zu ihren Enden hin verbreitert ist.

3. Röntgenuntersuchungsgerät nach Anspruch 1, dadurch gekennzeichnet, daß eine Blendenplatte (28) in den Strahlengang zwischen Röntgenröhre (6) und Patientenlagerungsplatte (3) eingesetzt ist, die auf die einzelnen Detektorelemente (10, 11, 12, 13, 14) der Detektoranordnung ausgereichtete Löcher (29) trägt.

4. Röntgenuntersuchungsgerät nach Anspruch 3, dadurch gekennzeichnet, daß die Blendenplatte (28) in Strahlenrichtung unmittelbar hinter dem Hohlzylinder (21) eingesetzt ist.

5. Röntgenuntersuchungsgerät nach Anspruch 3, dadurch gekennzeichnet, daß die Detektoranordnung (9) eine Reihe dicht nebeneinander angeordnete Einzeldetektoren (10, 11, 12, 13, 14) in der vom Strahlenbündel (9) überstrichenen Fächerebene umfaßt.

6. Röntgenuntersuchungsgerät nach Anspruch 1, dadurch gekennzeichnet, daß die Röntgenröhre (6) die Primärstrahlenausblendvorrichtung (7) und die Detektoranordnung (9) gemeinsam mit konstanter Geschwindigkeit quer zur Symmetrieachse (22) des Hohlzylinders (21) und zur Strahlenrichtung relativ zur Patientenlagerungsplatte (3) verschiebbar sind.

7. Röntgenuntersuchungsgerät nach Anspruch 6, dadurch gekennzeichnet, daß der Antrieb für die Verschiebung der Patientenlagerungsplatte (3) relativ zum übrigen Röntgenuntersuchungsgerät (1) und der Antrieb (15) für den Hohlzylinder (21) miteinander im Sinne einer dichten zeilenförmigen Abtastung gekuppelt sind.

8. Röntgenuntersuchungsgerät nach Anspruch 7, dadurch gekennzeichnet, daß sich die Patientenlagerungsplatte (3) bei jeder Umdrehung des Hohlzylinders (21) in etwa um die Breite des Strahlenbündels (8) relativ gegenüber dem übrigen Röntgenuntersuchungsgerät (1) verschiebt.

9. Röntgenuntersuchungsgerät nach Anspruch 1, dadurch gekennzeichnet, daß die Schlitze (25, 26) auf der Abwicklung (24) des Hohlzylinders (21) einen Winkel von 45° mit der Seitenkante der Abwicklung bilden.

## Claims

1. X-ray examination apparatus having a patient supporting plate (3), an X-ray tube (6) arranged on one side of the patient supporting plate (3), a primary radiation stop-down device (7) which produces a narrowly stopped X-ray beam (8) which can be swivelled in a plane in the shape of a fan, and a detector arrangement (9) which is arranged on the other side of the patient supporting plate (3) and exposed to the beam (8), characterised in that the stop-down device (7) comprises a hollow cylinder (21) which is uniformly driven and has its axis of symmetry (22) aligned approximately at right angles to the direction of the radiation and whose wall (24), which is opaque to radiation, carries two spiral slots (25, 26) which are staggered by 180° ; that between the radiation source (23) and the patient supporting plate (3), the hollow cylinder (21) is positioned at the end of a funnel-like holder (16) corresponding to the fan-shaped beam, so as to be remote from the focal point, the cross-section of which holder (16) is filled by the hollow cylinder (21) ; and that in the plane represented by the focal point (23) and the axis of symmetry (22) of the hollow cylinder (21), an additional slot-shaped focussing device (18, 20) is arranged close to the focal point.

2. X-ray examination apparatus as claimed in Claim 1, characterised in that the slot width is widened from the centre of the slot towards its ends.

3. X-ray examination apparatus as claimed in Claim 1, characterised in that a diaphragm plate (28) is inserted into the light path between X-ray tube (6) and the patient supporting plate (3) which carries apertures (29) which are aligned with the individual detector elements (10, 11, 12, 13, 14) of the detector arrangement.

4. X-ray examination apparatus as claimed in Claim 3, characterised in that the diaphragm plate (28) is inserted immediately behind the hollow cylinder (21) in the radiation direction.

5. X-ray examination apparatus as claimed in Claim 3, characterised in that the detector arrangement (9) comprises a line of individual detectors (10, 11, 12, 13, 14), which are arranged close beside one another, in the fan plane which is swept over by the beam (9).

6. X-ray examination apparatus as claimed in Claim 1, characterised in that the X-ray tube (6), the primary radiation stop-down device (7) and the detector arrangement (9) can be commonly displaced at a constant speed transversely to the axis of symmetry (22) of the hollow cylinder (21) and to the radiation direction, relative to the patient supporting plate (3).

7. X-ray examination apparatus as claimed in Claim 6, characterised in that the drive for moving the patient supporting plate (3) relative to the remainder of the X-ray examination apparatus (1) and the drive (15) for the hollow cylinder (21) are coupled to one another to provide close rectilinear scanning.

8. X-ray examination apparatus as claimed in Claim 7, characterised in that with each rotation of the hollow cylinder (21) the patient supporting plate (3) is displaced by approximately the width of the beam (8), relative to the remainder of the X-ray examination apparatus (1).

9. X-ray examination apparatus as claimed in Claim 1, characterised in that on the winding member (24) of the hollow cylinder (21) the slots (25, 26) form an angle of 45° with the lateral edge of the winding member.

**Revendications**

1. Appareil d'examen radiologique comportant un plateau (3) formant couchette pour patient, un tube à rayons X (6) situé d'un côté du plateau (3) formant couchette pour patient, un dispositif (7) de collimation du rayonnement primaire, produisant un faisceau de rayons X (8) étroitement collimaté et pouvant pivoter en forme d'éventail dans un plan, et un dispositif de détection (9) disposé d'un côté du plateau (3) formant couchette pour patient et soumis à l'action du faisceau de rayonnement (8), caractérisé par le fait que le dispositif de collimation (7) comporte un cylindre creux (21) dont l'axe de symétrie (22) est approximativement perpendiculaire à la direction du rayonnement et qui est entraîné de façon uniforme et dont la paroi (24) opaque au rayonnement comporte deux fentes (25, 26) de forme hélicoïdale, décalées de 180° l'une par rapport à l'autre, que le cylindre creux (21) est monté entre la source de rayonnement (23) et le plateau (3) formant couchette pour patient, dans une position éloignée du foyer, à l'extrémité d'un support (16) en forme de trémie, qui correspond au faisceau de rayonnement en forme d'éventail et dont la section transversale est remplie par le cylindre creux (21), et qu'un dispositif supplémentaire de collimation en forme de fente (18, 20) proche du foyer, est disposé dans le plan déterminé par le foyer (23) et par l'axe de symétrie (22) du cylindre creux (21).

2. Appareil d'examen radiologique suivant la revendication 1, caractérisé par le fait que la largeur de la fente augmente depuis le milieu de cette dernière en direction de ses extrémités.

3. Appareil d'examen radiologique suivant la revendication 1, caractérisé par le fait qu'un volet de diaphragme (28), qui comporte des trous (29) alignés sur les différents éléments détecteurs (10, 11, 12, 13, 14) du dispositif de détection, est inséré sur le trajet du rayonnement entre le tube à rayons X (6) et le plateau (3) formant couchette pour patient.

4. Appareil d'examen radiologique suivant la revendication 3, caractérisé par le fait que le volet de diaphragme (28) est inséré, sur le trajet du rayonnement, directement en arrière du cylindre creux (21).

5. Appareil d'examen radiologique suivant la revendication 3, caractérisé par le fait que le dispositif de détection (9) comporte une série de détecteurs individuels (10, 11, 12, 13, 14), disposés en étant serrés les uns à côté des autres, dans le plan de l'éventail, qui est balayé par le faisceau de rayonnement (9).

6. Appareil d'examen radiologique suivant la revendication 1, caractérisé par le fait que le tube à rayons X (6), le dispositif (7) de collimation du rayonnement primaire et le dispositif de détection (9) peuvent être déplacés en commun, à une vitesse constante, transversalement à l'axe de symétrie (22) du cylindre creux (21) et à la direction du rayonnement, par rapport au plateau (3) formant couchette pour patient.

7. Appareil d'examen radiologique suivant la revendication 6, caractérisé par le fait que le dispositif d'entraînement servant à réaliser le déplacement du plateau formant couchette pour patient par rapport au reste de l'appareil (1) d'examen radiologique et le dispositif (15) d'entraînement du cylindre creux (21) sont accouplés entre eux en vue d'obtenir un balayage suivant des lignes étroitement rapprochées les unes des autres.

8. Appareil d'examen radiologique suivant la revendication 7, caractérisé par le fait que le plateau (3) formant couchette pour patient se déplace, lors de chaque rotation du cylindre creux (21), sur une distance égale approximativement à la largeur du faisceau de rayonnement (8), par rapport au reste de l'appareil (1) d'examen radiologique.

9. Appareil d'examen radiologique suivant la revendication 1, caractérisé par le fait que les fentes (25, 26) font, sur la projection développée (24) du cylindre creux (21), un angle de 45° avec le bord latéral de cette projection développée.

FIG 1

1

FIG 2

FIG 3

FIG 4